(19) Europäisches Patentamt
European Patent Office
Office européen des brevets

(11) Publication number : 0 330 613 B1

# (12) EUROPEAN PATENT SPECIFICATION

(45) Date of publication of patent specification : 18.08.93 Bulletin 93/33

(51) Int. Cl.$^5$ : C23F 11/16, C23F 11/14, C09D 5/08, C07C 323/00, C10M 135/20

(21) Application number : 89810120.9

(22) Date of filing : 16.02.89

## (54) Compositions and method for inhibiting corrosion.

(30) Priority : 25.02.88 GB 8804457

(43) Date of publication of application : 30.08.89 Bulletin 89/35

(45) Publication of the grant of the patent : 18.08.93 Bulletin 93/33

(84) Designated Contracting States : BE DE FR GB IT

(56) References cited :
FR-A- 1 032 470
PATENT ABSTRACTS OF JAPAN, vol. 8, no. 225 (C-247)[1662], 16th October 1984; & JP-A-59 108 097

(56) References cited :
CHEMICAL ABSTRACTS, vol. 100, part 225, 25th June 1984, page 43, abstract no. 211057v, Columbus, Ohio, US; & JP-A-59 006 252
CHEMICAL ABSTRACTS, vol. 101, part 22, 26th November 1984, page 167, abstract no. 194967t, Columbus, Ohio, US; & JP-A-59 108 099

(73) Proprietor : CIBA-GEIGY AG
Klybeckstrasse 141
CH-4002 Basel (CH)

(72) Inventor : Phillips, Emyr, Dr.
1 Hill Top View Tingley
Wakefield West Yorkshire (GB)
Inventor : Wasson, Robert Craig, Dr.
27 Phythian Crescent Penketh
Warrington Cheshire WA5 2BT (GB)

EP 0 330 613 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to new compositions, in particular to compositions comprising a coating material and a corrosion-inhibiting aromatic or heterocyclic compound containing a phenolic substituent.

In European Patent Specification No. 3817A, 2-mercaptobenzothiazole and its salts are disclosed as corrosion inhibitors. It is also known that various derivatives of 2-mercaptobenzothiazole exhibit corrosion-inhibiting properties. For example, European Patent Specification 129506A discloses benzothiazol-2-yl thiocarboxylic acids containing hydrophilic groups and their use as corrosion inhibitors.

We have now found, surprisingly, that certain aromatic or heterocyclic compounds containing a phenolic substituent carrying hydrophobic groups have, in addition to excellent corrosion-inhibiting properties, activity as antioxidants in coating materials.

Accordingly, the present invention provides a composition comprising a coating material and, at least one compound having the formula I:

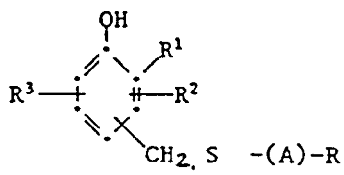

I

wherein $R^1$ and $R^2$ may be the same or different and each is hydrogen, $C_1$-$C_{15}$alkyl, $C_3$-$C_{15}$alkenyl, $C_7$-$C_{10}$phenylalkyl, $C_6$-$C_{18}$aryl having 6 or 10 aromatic ring carbon atoms; or $C_5$-$C_{12}$cycloalkyl;

$R^3$ is hydrogen, $C_1$-$C_{15}$alkyl or $C_2$-$C_{15}$alkenyl;

A is a residue of a heterocyclic ring having 5-7 ring members and 1-4 nitrogen atoms, or A is a residue of a heterocyclic ring having 5-7 ring members and 1-4 nitrogen atoms and is fused to a further heterocyclic ring or to a carbocyclic ring,

and wherein R is one or more of hydrogen, $C_1$-$C_{15}$alkyl, $C_1$-$C_4$halogenoalkyl, $C_1$-$C_{12}$alkoxy, $C_1$-$C_{12}$alkylthio, phenylthio, benzylthio, $C_1$-$C_{12}$alkylsulphonyl, phenyl, $C_7$-$C_{15}$alkylphenyl, $C_7$-$C_{10}$phenylalkyl, $C_5$-$C_8$cycloalkyl, halogen, $NO_2$, CN, COOH, COO($C_1$-$C_{12}$alkyl), OH, $NH_2$, $CONH_2$, $NHR^4$, $N(R^4)_2$, $CONH(R^4)$ or $-CON(R^4)_2$ wherein $R^4$ is $C_1$-$C_{12}$alkyl, $C_3$-$C_{12}$alkyl which is interrupted by one or more O-atoms, $C_5$-$C_8$cycloalkyl, benzyl, phenyl or phenyl which is substituted by halogen, $C_1$-$C_4$alkyl, $C_1$-$C_4$alkoxy or $NO_2$, or $N(R^4)_2$ is a pyrrolidino, piperidino or morpholine group; provided that A is not benzothiazole residue.

When A is the residue of an N-containing 5-7 membered heterocycle or a heterocycle fused to a heterocyclic or carbocyclic ring other than a benzothiazole residue, it may contain one or more heteroatoms especially oxygen and sulphur atoms and may contain a substituent having the formula -C=X in which X is oxygen or sulphur, preferably sulphur.

When R, $R^1$, $R^2$, $R^3$ and $R^4$ are $C_1$-$C_{15}$alkyl such alkyl groups may be branched or unbranched. Specific examples of such alkyl groups include methyl, ethyl, n-propyl, isopropyl, n-butyl, sec-butyl, isobutyl, tert.butyl, pentyl, hexyl, n-octyl, 2-ethylhexyl, 1,1,3,3-tetramethylbutyl, 1,1,3,3,5,5-hexamethylhexyl, n-decyl, isodecyl, n-dodecyl and pentadecyl groups. $R^3$ may be preferably $C_1$-$C_4$alkyl groups and may be methyl, ethyl, n-propyl, isopropyl, n-butyl, sec.-butyl, isobutyl or tert.-butyl groups. Especially preferred for $R^3$ are hydrogen, methyl or pentadecyl groups. $R^3$ may be $C_2$-$C_{15}$alkenyl and preferably a pentadecenyl group.

$C_7$-$C_{10}$phenylalkyl groups $R^1$, $R^2$ and R may be benzyl, 1-phenylethyl, 2-phenylethyl, α,α-dimethylbenzyl or 2-phenylpropyl groups.

Halogenoalkyl groups R may be e.g. chloromethyl, trichloromethyl, bromomethyl, 2-chloroethyl, 2,2,2-trichloromethyl, trifluoromethyl or 2,3-dichloropropyl.

As alkoxy, alkylthio or alkylsulphonyl, R may be methoxy, ethoxy, isopropoxy, butoxy, hexyloxy, octyloxy, dodecyloxy, methylthio, ethylthio, ethylthio, nonylthio, methylsulphonyl, ethylsulphonyl, hexylsulphonyl or dodecylsulphonyl.

Alkylphenyl groups R include tolyl, xylyl, 4-ethylphenyl, 4-tert.butylphenyl, 4-octylphenyl or 4-nonylphenyl. $C_5$-$C_8$cycloalkyl groups R or $R^4$ include cyclopentyl, cycloheptyl, methylcyclohexyl or cyclooctyl.

When $R^4$ is phenyl which is substituted by halogen, $C_1$-$C_4$alkyl, $C_1$-$C_4$alkoxy or $NO_2$, $R^4$ may be 4-chlorophenyl, 3-bromophenyl, 2-fluorophenyl, p-tolyl, 3,5-dimethylphenyl, 4-isopropylphenyl, 4-methoxyphenyl, 3-ethoxyphenyl, 4-nitrophenyl or 4-nitro-2-methylphenyl.

When $R^4$ is $C_3$-$C_{12}$alkyl interrupted by one or more oxygen atoms, it may be 2-methoxyethyl, 2-butoxyethyl, 3,6-dioxaheptyl or 3,6-dioxadecyl.

N-containing heterocyclic residues A are mono- or bi-cyclic; when A is a bicyclic residue it consists of a heterocyclic ring fused to a further heterocyclic ring or to a carbocyclic ring.

Examples of optionally substituted monocyclic heterocyclic residues A include 1,2,4-triazole, imidazole, pyrazole, tetrazole, thiazolin-2-thione, imidazolin-2-thione, N-methyl-imidazolon-2-thione and 5-(3-phenyl-1,3,4-thia-diazol-2(3H)-thione), 2-pyridine, 4-pyridine, 3-pyridazine, 2-pyrimidine, 2-thiazole, 2-thiazoline, 3-(1,2,4-triazole) and 5-(2-mercapto-1,3,4-thiadiazole).

Examples of bicyclic, all-heterocyclic residues A are naphthyridine, purine and pteridine residues, while bicyclic heterocyclic-aromatic residues A include benzimidazole, benzotriazole, benzoxazolin-2-thione and 2-benzoxazole.

Preferred compositions according to the present invention include those containing a compound of formula I containing a residue A in which one, two or three R's and preferably one R, have their previous significance and the other R's are hydrogen.

Preferred compositions according to the present invention include those containing a compound of formula I containing a residue A in which one R is hydrogen, $C_1$-$C_4$alkyl, $C_1$-$C_4$alkoxy, trifluoromethyl, halogen or nitro and the other R's are hydrogen; $R^1$ is $C_1$-$C_8$alkyl, $C_7$-$C_{10}$phenylalkyl, phenyl or cyclohexyl; $R^2$ is hydrogen, $C_1$-$C_8$alkyl, $C_7$-$C_{10}$phenylalkyl, phenyl or cyclohexyl; and $R^3$ is hydrogen.

With respect to the compounds of formula I, the phenolic group is preferably positioned in the para- or ortho-position relative to the $-CH_2$- S -(A)-R group.

When the phenolic group is in the para-position to the $-CH_2$-S-(A)-R group, preferred compounds are those having the formula IA:

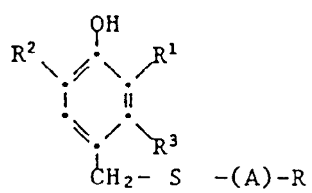

IA

wherein R, $R^1$, $R^2$, $R^3$, n and A have their previous significance.

Preferred compounds of formula IA are those in which $R^1$ and $R^2$, independently, are hydrogen, $C_1$-$C_{15}$alkyl, $C_7$-$C_{10}$phenylalkyl, phenyl or cyclohexyl, and $R^3$ is hydrogen or methyl; more preferred compounds of formula IA are those wherein $R^1$ and $R^2$ are $C_1$-$C_5$alkyl and $R^3$ is hydrogen.

When the phenolic group is in the ortho-position to the $-CH_2$-S-(A)-R group, preferred compounds are those having the formula IB:

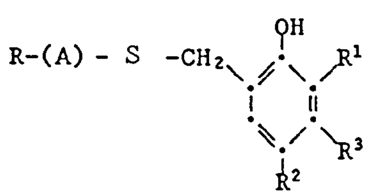

IB

wherein R, $R^1$, $R^2$, $R^3$, n and A have their previous significance.

Preferred compounds of formula IB are those wherein $R^1$ is $C_1$-$C_8$alkyl, $C_1$-$C_4$alkoxy, $C_7$-$C_{10}$phenylalkyl, phenyl or cyclohexyl, and $R^2$ is hydrogen $C_1$-$C_8$alkyl, $C_7$-$C_{10}$phenylalkyl, phenyl, or cyclohexyl and $R^3$ and A have their previous significance.

More preferred compounds of formula IB are those wherein $R^1$ and $R^2$ are each $C_1$-$C_5$alkyl and $R^3$ and A have their previous significance.

Specific examples of compounds of formula IA in which A is the residue of a monocyclic heterocyclic ring are summarised in the following Table 1:

Table 1

| A | R | $R^1$ | $R^2$ | $R^3$ |
|---|---|---|---|---|
| 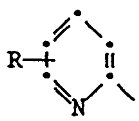 | H<br>H<br>H<br>3-$CO_2H$<br>H | $^{t}Bu$<br>$^{t}Bu$<br>$^{t}Bu$<br>$^{t}Bu$<br>Me | $^{t}Bu$<br>Me<br>Pr<br>$^{t}Bu$<br>Me | H<br>H<br>H<br>H<br>Me |
| 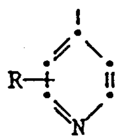 | H<br>H<br>H<br>H<br>H | Ph<br>$^{t}Bu$<br>Me<br>Me<br>$^{t}Bu$ | Ph<br>$^{t}Bu$<br>Me<br>Me<br>Me | H<br>H<br>H<br>Me<br>H |
| 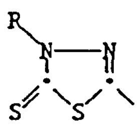 | H<br>Ph<br>Ph<br>Ph | $^{t}Bu$<br>$^{t}Bu$<br>Me<br>Me | $^{t}Bu$<br>$^{t}Bu$<br>Me<br>Me | H<br>H<br>Me<br>H |
| 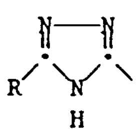 | H<br>n$C_4H_9$ | $^{t}Bu$<br>$^{t}Bu$ | $^{t}Bu$<br>$^{t}Bu$ | H<br>H |

Specific examples of compounds of formula IB in which A is a residue of a heterocyclic ring are summarised in Table 2.

Table 2

| A | R | $R^1$ | $R^2$ | $R^3$ |
|---|---|---|---|---|
| 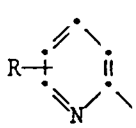 | H<br>H<br>3-$CO_2H$<br>3-$CO_2H$ | $^{t}Bu$<br>Me<br>$^{t}Bu$<br>$^{t}Bu$ | $^{t}Bu$<br>Me<br>$^{t}Bu$<br>Me | H<br>H<br>H<br>H |
| 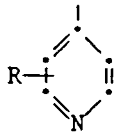 | H<br>H<br>H | $^{t}Bu$<br>$^{t}Bu$<br>$^{i}Br$ | $^{t}Bu$<br>Me<br>$^{i}Pr$ | H<br>H<br>H |

Those compounds of formula I and having the sub-generic formula:

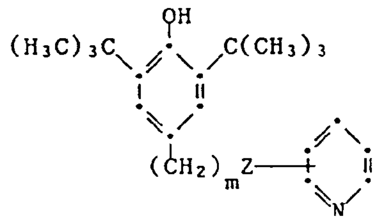

wherein Z is inter alia S and m is 0, 1 or 2 are known, and have been disclosed as antichloesteremics, hypolipemics and cerebal vasodilators in German Offenlegungsschrift 2,726,125.

Also known are those compounds of formula I have the sub-generic formulae:

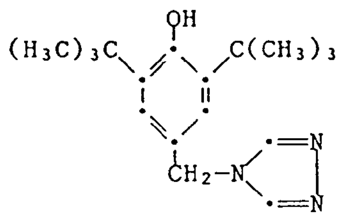

or

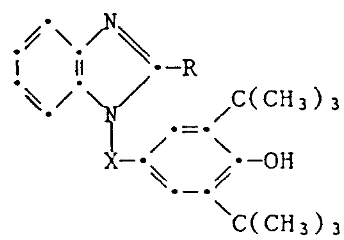

wherein X is $CH_2$ or $SO_2$; and R is hydrogen, methyl or $-CH_2OH$.

These compounds are disclosed as antioxidants in Rev. Roum. Chim. 1983, 28, 129-37.

Still further compounds of formula I having the sub-generic structure:

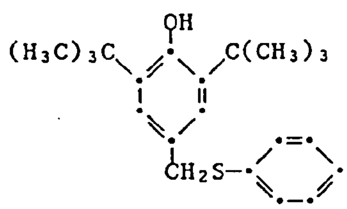

have been described as stabilisers and antioxidants for olefin polymers in US 3,637,863.

All the other compounds of the formula I are novel compounds and, as such, form a further aspect of the present invention.

Accordingly the present invention also provides compounds having the formula I'

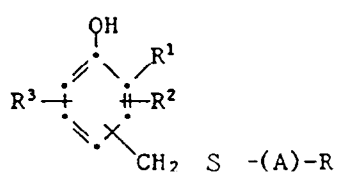

I'

wherein R, $R^1$, $R^2$, $R^3$, n and A have their previous significance, provided that A is not benzothiazole residue; and

when A is a heterocyclic residue having 5-7 ring members and 1-4 nitrogen atoms, $R^2$ is H, $R^1$ and $R^3$ are each tert.-butyl and $R^3$ is in ortho-position to the -OH group, then A is not pyridino-thio group.

The compounds of formula (I or I') may be prepared by reacting in the presence of an acid catalyst a phenol compound having the formula II:

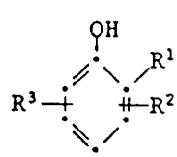

(II)

having at least one free position ortho- or para to the -OH group, wherein $R^1$, $R^2$ and $R^3$ have their previous significance, with formaldehyde and a precursor compound for the group A, wherein A has its previous significance.

The precursor compound for the group of formula A may be, for example, compound R-(A)-SH wherein R and A have their previous significance.

It will be appreciated that when the precursor compound has the formula R-(A)-SH then the residue R-(A)- in the compound of formula I so obtained may either be in the sulphide or thione form, the former being convertible into the latter by heating.

Heating can be carried out with or without a solvent.

Examples of suitable solvents are aromatic hydrocarbons, such as toluene or xylene; halogenated hydrocarbons, such as tetra-chloroethylene or chlorobenzene; alkanols, such as isopropanol or n-butanol; or esters, ketones, dimethylformamide or dimethyl sulfoxide. Polar solvents, for example dimethylformamide, accelerate the reaction. The rearrangement can also be accelerated by adding basic catalysts. Examples of the latter are, in particular, aliphatic, cycloaliphatic or heterocyclic amines. If the phenolic OH group is in the para-position relative to the radical $-CH_2-$, the rearrangement proceeds more rapidly than if the group is in the ortho-position. The temperature required for the rearrangement therefore depends on the position of the OH group and on the solvent and catalyst used. It is preferably carried out at 70-250°C, in particular at 100-200°C.

The compounds of formula (I or I') may also be prepared by reacting a phenol compound having the formula III:

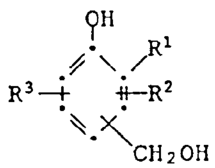

III

wherein $R^1$, $R^2$ and $R^3$ have their previous significance, with a precursor compound for the group R-(A )-S-, e.g. a compound having the formula R-(A)-SH.

A further process for the production of compounds of formula (I or I') comprises reacting a phenol compound of formula IV:

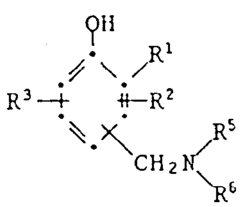

(IV)

wherein $R^1$, $R^2$ and $R^3$ have their previous significance and $R^5$ and $R^6$, independently, are $C_1$-$C_{12}$alkyl, $C_5$-$C_8$cycloalkyl, benzyl or phenyl, with a compound R-(A)-SH.

The reaction is preferably effected at elevated temperature e.g. 50-200°C, especially 70-150°C and in the presence of a polar organic solvent e.g. a $C_1$-$C_4$alcohol, dimethylformamide or dimethylsulphoxide.

Another method for producing compounds of formula (I or I') comprises reacting in the presence of a basic catalyst, a phenol compound having the formula II:

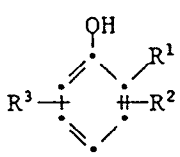

II

wherein $R^1$, $R^2$ and $R^3$ have their previous significance. with formaldehyde, and with a precursor compound for the group R-(A)-S-, e.g. a compound R-(A)-SH

In this process also, isomeric compounds may be produced and conversion of sulphide compounds into thione compounds by heating, may be effected as indicated hereinbefore.

The reaction may be performed with or without solvent and preferably within the temperature range of 70-150°C.

Any strong base may be used as catalyst, preferred examples include primary-, secondary- or tertiary amines such as isopropylamine, butylamine, cyclohexylamine, dibutylamine, dihexylamine, diisopropylamine, triethylamine, tributylamine, piperidine, morpholine, pyrrolidone or quinoline.

The formaldehyde used for the reaction may be, for example, an aqueous solution (formalin) in the form of paraformaldehyde, or a compound which releases formaldehyde, under the reaction conditions, for example hexa-methylene tetramine.

The compounds for the formula I are effective as corrosion inhibitors and as antioxidants. As such, they can be added to any liquid or solid coating materials.

Examples of coating materials are lacquers, paints or varnishes. They always contain a film-forming binder as well as other optional components.

Examples of coating materials are materials based on a epoxide, polyurethane, aminoplast, acrylic, alkyd or polyester resin and on mixtures of such resins. Further examples of suitable binders are vinyl resins, such as polyvinyl acetate, polyvinylbutyral, polyvinyl chloride and copolymers thereof, cellulose esters, chlorinated rubbers, phenolic resins, styrenelbutadiene copolymers and drying oils.

The coating materials can contain solvents or can be free from solvents or they can be aqueous systems (dispersions, emulsions or solutions). They can be pigmented or non-pigmented and they can be also be metallized. In addition to the corrosion inhibitors according to the invention, they can contain other additives customary in the technology of coating materials, for example fillers, flow control auxiliaries, dispersing auxiliaries, thixotropic agents, adhesion promoters, antioxidants, light stabilizers or curing catalysts. They can also contain other known anti-corrosion agents, for example anti-corrosion pigments, such as pigments containing phosphates or borates, or metal oxide pigments, or other organic or inorganic corrosion inhibitors, for example salts of nitroisophthalic acid, phosphorus esters, technical amines or substituted benzotriazoles.

It is also advantageous to add basic fillers or pigments which, in certain binder systems, produce a synergistic effect on the inhibition of corrosion. Examples of such basic fillers and pigments are calcium carbonate, magnesium carbonate, zinc oxide, zinc carbonate, zinc phosphate, magnesium oxide, aluminium oxide, aluminium phosphate or mixtures thereof. Examples of basic organic pigments are pigments based on aminoanthraquinone.

The corrosion inhibitor can also be applied to a carrier. Pulverulent fillers or pigments are particularly suitable for this purpose. This technique is described in greater detail in German Patent A 3,122,907.

The corrosion inhibitors can be added to the coating material during its preparation, for example during the dispersion of pigments by grinding, or the inhibitor is dissolved in a solvent beforehand and the solution is stirred into the coating agent. The inhibitor is used in an amount of 0.1 to 20 % by weight, preferably 0.5 to 5 % by weight, based on the solids content of the coating material.

The coating materials can be applied to the substrate by the customary process, for example by spraying, dipping, brushing or by electrodeposition, in particular cathodic dip-coating. Several layers are often applied. The corrosion inhibitors are added primarily to the base layer, since they act particularly on the metal/coating interface. It is also possible, however, additionally to add the inhibitors to the top layer or intermediate layer, where they are available as a depot. Depending on whether the binder is a physically drying resin or a heat-curable or radiation-curable resin, the coating is cured at room temperature or by heating (stoving) or by irradiation.

The coating material is preferably a primer for metallic substrates e.g iron, steel, copper, zinc or aluminium. The coating material can be an aqueous system especially a cathodically-depositable paint (cataphores lacquer).

It is a particular advantage of the compounds of the formula I that they do not tend to become chalky. It is a further advantage that they have a favourable effect on the adhesion of coating to metal and, finally, that they exert an antioxidative action on the coating and thus reduce the chalking of pigments and fillers. All these properties contribute towards prolonging the useful life of the coating.

It can be important to add a mixture of several compounds of the formula I. For example, if certain technical mixtures of phenols are used in the preparation of the compounds of the formula I, a mixture of products of the formula I is inevitably formed, and this can be used as such. However, in order to lower the melting point it can also be advantageous to mix two or more of such compounds.

Relative to known corrosion inhibitors, the compounds of formula I exhibit lower water absorption, chemical inertness and higher stability to heat.

The peparation and use of compounds of the formula I are described in greater detail in the following Examples. In these parts and percentages are by weight, unless stated otherwise. The temperatures is quoted in °C.

Example 1: (3,5-Ditertbutyl-4-hydroxybenzyl)-4-mercaptopyridine

4-Mercaptopyridine(11.1 g, 0.1 mol), 2,6-di-tertbutylphenol (20.6 g, 0.1 mol), paraformaldehyde (3.0 g, 0.1 M) and di-n-butylamine (1 ml) are heated at 80°C for two and half hours, after which time, thin layer chromatography (t.l.c.) showed the reaction to be complete. The reaction mixture is cooled to 50°C, ethanol (100 ml) is added and the product is crystallised as a pale yellow solid (24.7 g, 78.4 % of theory) of m.p. 169-171°C (Found C = 73.30; H = 8.49; N = 4.31; S = 9.71 $C_{20}H_{27}NOS$ requires C = 72.95; H = 8.21; N = 4.26 and S = 9.73 %).

Example 2: (3,5-Di-tert-butyl-4-hydroxybenzyl)-3-mercapto-1,2,4-triazole

3-Mercapto-1,2,4-triazole (20.2 g, 0.2 mol), 2,6-di-tertbutylphenol (41.2 g, 0.2 mol), paraformaldehyde (6.0 g, 0.2 M) and di-n-butylamine (2 ml) are heated, under reflux, in ethanol (100 ml) for ten hours. The reaction mixture is cooled, and the product is crystallised as a white solid (43 g, 67 % of theory) having a m.p. of 184-185°C (Found C = 63.39; H = 8.03; N = 13.29; S = 10.20 $C_{17}H_{25}N_3OS$ requires C = 63.95; H = 7.84; N = 13.17 and S = 10.03 %).

Example 3: (3,5-Di-tert-butyl-4-hydroxybenzyl)-5-mercapto-3-phenyl-1,3,4-thiadiazole-2(3H) thione

5-Mercapto-3-phenyl-1,3,4-thiadiazole-2(3H) thione potassium salt (26.4 g, 0.1 mol), 2,6-di-tertbutylphenol (20.6 g, 0.1 mol), formalin (11 ml) and concentrated sulphuric acid (10 ml) in ethanol (100 ml) are heated, under reflux, for six hours. After this time, the ethanol is removed by rotary evaporation, and partitioned between ethyl acetate and water. The organic phase is dried over magnesium sulphate, filtered and the solvent removed in vacuo. A yellow oil is left (29.5 g, 66 % of theory).

Example 4: (3,5-Di-tert-butyl-4-hydroxybenzyl)-3-thiazoline-2-thione

2-Mercaptothiazoline (23.8 g, 0.2 mol), 2,6-di-tertbutylphenol (20.6 g, 0.2 mol), paraformaldehyde (6 g, 0.2 mol) and di-n-butylamine (1 ml) are heated, under nitrogen, at 180°C for two hours. After this time, the reaction is,cooled to 70° and ethanol (200 ml) is added. The product crystallises as a white solid (48 g, 71 % of theory) of m.p. 138-140°C (Found C = 63.95; H = 8.14; N = 4.13; S = 18.61 $C_{18}H_{27}NOS_2$ requires C = 64.09; H = 8.01; N = 4.15; S = 18.99 %).

Example 5: (3-Tertbutyl-5-methyl-4-hydroxybenzyl)-benzoxazolin-2-thione

2-Mercaptobenzoxazole (10.96 g, 0.073 mol), 2-tertbutyl-6-methylphenol (11.9 g, 0.073 mol), paraformaldehyde (2.18 g, 0.073 mol) and di-n-butylamine (0.5 ml) are heated to 120°C. After two hours, the reaction mixture solidifies. The solid mass is crystallised from ethanol to yield a white solid (19.8 g, 84 % of theory) of m.p. 155-157°C (Found N = 4.17; $C_{19}H_{21}NO_2S$ requires N = 4.28 %).

Examples 6 to 26:

Using the procedure described in Example 1, the compounds summarised in the following Table 1 are prepared:

Examples indicating IA are those having the formula IA as described above, examples indicating IB are those having formula IB as described above.

Table I

| Example | A | R | $R_1$ | $R_2$ | $R_3$ | |
|---|---|---|---|---|---|---|
| 6 | 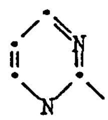 | H | t-butyl | t-butyl | H | IA |
| 7 | 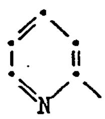 | H | t-butyl | t-butyl | H | IA |
| 8 | 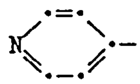 | H | t-butyl | methyl | H | IB |
| 9 | 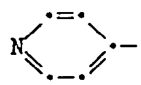 | H | methyl | methyl | methyl | IA |
| 10 | 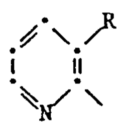 | $CO_2H$ | t-butyl | t-butyl | H | IA |
| 11 | 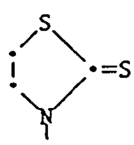 | H | H | H | $C_{15}H_{27}$ | IB |
| 12 | 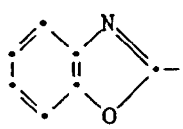 | H | t-butyl | methyl | H | IB |
| 13 | 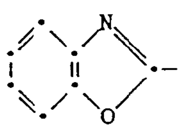 | H | methyl | methyl | methyl | IB |
| 14 | 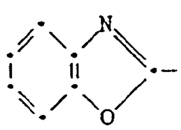 | H | t-butyl | t-butyl | H | IB |

Table I (continuation)

| Example | A | R | $R_1$ | $R_2$ | $R_3$ | |
|---|---|---|---|---|---|---|
| 15 | 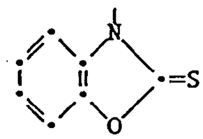 | H | H | H | $C_{15}H_{27}$ | IB |
| 16 | 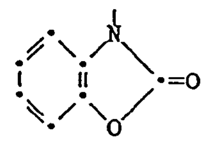 | H | t-butyl | t-butyl | H | IA |
| 17 | 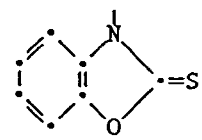 | H | isopropyl | isopropyl | H | IA |
| 18 | 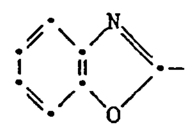 | H | isopropyl | isopropyl | H | IB |
| 19 | 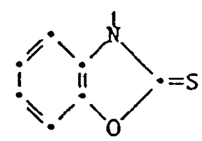 | H | methyl | methyl | methyl | IB |
| 20 | 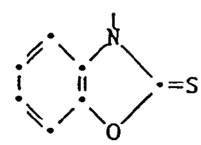 | H | t-butyl | t-butyl | H | IA |
| 21 | 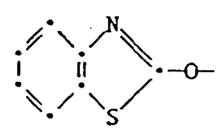 | H | t-butyl | methyl | H | IB |
| 22 | 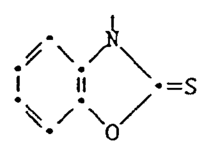 | H | methyl | t-butyl | H | IA |
| 23 | 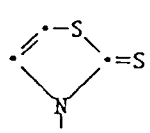 | H | t-butyl | t-butyl | H | IA |

Table I (continuation)

| Example | A | R | $R_1$ | $R_2$ | $R_3$ | |
|---|---|---|---|---|---|---|
| 24 | 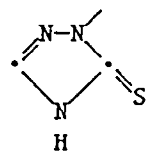 | H | t-butyl | t-butyl | H | IA |
| 25 | 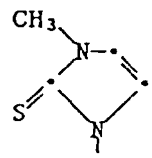 | H | t-butyl | t-butyl | H | IA |
| 26 | 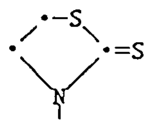 | H | t-butyl | t-butyl | H | IA |

Analytical data relating to the compounds of Examples 6 to 26 are set out in the following Table IA:

Table IA

| Example | Mpt°C | Analysis |
|---|---|---|
| 6 | 132-4 | 'HNMR ($CDCl_3$) δ 1.95 (18H), δ 4.25 (2H), δ 6.75 (1H), δ 7.10 (2H), δ 8.30 (2H) Elemental analysis C, 69.52; H, 8.04; N, 8.51; S, 9.59 % Theory C, 69.09; H, 7.89; N, 8.48; S 9.70 % |
| 7 | 98-9 | 'HNMR ($CDCl_3$) δ 1.41 (18H), δ 4.35 (2H), δ 6.75 (1H), δ 6.85 (1H), δ 7.10 (2H) δ 7.25 (1H), δ 8.25 (1H) Elemental analysis C, 73.25; H, 8.29; N, 4.32 % Theory C, 73.95; H, 8.21; N, 4.26 % |
| 8 | 187-8 | 'HNMR ($DMSO^{d6}$) δ 1.38 (9H), δ 2.15 (3H) δ 4.35 (2H), δ 7.0 (2H), δ 7.35 (2H), δ 8.39 (2H) Elemental analysis C, 70.09; H, 7.38; N, 4.88; S, 11.15 % Theory C, 71.08; H, 7.32; N, 4.85; S, 11.41 % |
| 10 | 208-9 | 'HNMR ($DMSO^{d6}$) δ 1.35 (18H), δ 4.21 (2H), δ 6.92 (1H), δ 7.15 (2H), δ 8.10 (1H), δ 8.45 (1H) |
| 11 | - | 'HNMR ($CDCl_3$) δ 1.0-2.8 (27H), δ 3.2 (2H), δ 3.90 (2H), δ 5.4 (2H), δ 6.7 (1H), δ 7.0 (2H) |
| 12 | 84-5 | 'HNMR ($CDCl_3$) δ 1.39 (9H), δ 2.15 (3H), δ 4.35 (2H), δ 6.80 (2H), δ 7.0-7.5 (4H) |
| 13 | 174-7 | 'HNMR ($CDCl_3/DMSO^{d6}$) δ 2.17 (9H), δ 5.2 (2H), δ 6.9-7.4 (5H) |
| 14 | 115-9 | 'HNMR ($CDCl_3$) δ 1.3 (9H), δ 1.45 (9H), δ 4.4 (2H), δ 6.95-7.15 (5H) |
| 15 | viscous oil | 'HNMR ($CDCl_3$) δ 1.15 (21H), δ 2.0-3.0 (6H), δ 5.25 (2H), δ 6.5-7.5 (7H) |
| 16 | 136.5 - 137 | 'HNMR ($CDCl_3$) δ 1.19 (18H), δ 4.85 (2H), δ 6.9-7.1 (6H) |
| 17 | 117-8 | 'HNMR ($CDCl_3$) δ 1.15-1.25 (6H), δ 2.05 (2H), δ 5.19 (2H), δ 6.95 (6H) |

| Example | Mpt°C | Analysis |
|---|---|---|
| 18 | 97-9 | 'HNMR ($CDCl_3$) δ 1.15 (6H), δ 2.75 (2H), δ 3.20 (1H), δ 4.38 (2H), δ 6.85-7.60 (6H) |
| 19 | 209-212 | 'HNMR ($DMSO^{d6}$) δ 2.10 (9H), δ 5.15 (2H), δ 6.60-7.55 (5H)<br>Elemental analysis C, 67.18; H, 5.65; N, 4.92; S, 10.91 %<br>Theory C, 68.23; H, 5.69; N, 4.68; |
| 20 | 151-2 | 'HNMR ($CDCl_3$) δ 1.35 (18H), δ 5.20 (2H), δ 7.15 (6H)<br>Elemental analysis C, 71.54; H, 7.37; N, 3.75; S, 8.41 %<br>Theory C, 71.55; H, 7.32; N, 3.79; S. 8.67 % |
| 21 | 169-70 | 'HNMR ($CDCl_3$) δ 1.35 (9H), δ 2.20 (3H), δ 4.90 (2H), δ 6.90-7.30 (6H)<br>Elemental analysis C, 69.24; H, 6.36; N, 4.26; S, 9.21 %<br>Theory C, 69.72; H, 6.42; N, 4.28; S, 9.78 % |
| 22 | 155-7 | 'HNMR ($CDCl_3$) δ 1.19 (9H), δ 2.15 (3H), δ 5.20 (2H), δ 6.90-7.20 (6H)<br>Elemental analysis C, 68.50; H, 6.04; N, 4.17 %<br>Theory C, 69.72; H, 6.42; N, 4.28 % |
| 23 | - | 'HNMR ($CDCl_3$) δ 1.40 (18H), δ 5.05 (2H), δ 6.75 (2H), δ 6.95 (2H) |
| 24 | - | 'HNMR ($CDCl_3$) δ 1.40 (18H), δ 5.00 (2H), δ 6.98 (3H) |
| 25 | 128-9 | 'HNMR ($CDCl_3$) δ 1.38 (18H), δ 3.50 (3H), δ 5.00 (2H), δ 6.45 (2H), δ 7.0 (2H)<br>Elemental analysis C, 68.41; H, 8.73; N, 8.46; S, 9.90 %<br>Theory C, 68.67; H, 8.43; N, 8.43; S, 9.64 % |
| 26 | 138-140 | 'HNMR ($CDCl_3$) δ 1.40 (18H), δ 3.20 (2H), δ 4.00 (2H), δ 4.85 (2H), δ 7.15 (2H)<br>Elemental analysis C, 63.95; H, 8.14; N, 4.13; S, 18.61 %<br>Theory C, 64.09; H, 8.01; N, 4.15; S, 18.99 % |

<u>Examples 27 to 52:</u>

An alkyd resin paint is prepared in accordance with the following formulation:

40 parts of Alphthalate® 380 (60 % solution in xylene), alkyd resin made by Reichhold Albert Chemie AG,
10 parts of iron oxide red 224 made by Bayer AG,
13.6 parts of talc (micronized),
13 parts of micronized calcium carbonate (Millicarb®, Pluss-Staufer AG),

0.3 part of skin prevention agent Luaktin® 9 BASF),
0.6 part of 8 % solution of cobalt naphthenate and
22.5 parts of 6:40 xylene/ethyleneglycol mixture.

The paint is ground with glass beads to a pigment and filler particle size of 10-15 µm. The corrosion inhibitors indicated in the tables below are added before grinding.

The paint is sprayed into sand-blasted steel sheets measuring 7 x 13 cm in a layer thickness amounting to approximately 50 µm after drying. After drying at room temperature for 7 days, the samples are subjected to after-curing at 60° for 60 minutes.

Two cruciform cuts of length 4 cm are cut, down to the metal, in the cured paint surface by means of a Bonder cross-cut apparatus. The edges are protected by applying an edge-protection agent (Icosit® 255) to the latter.

The samples are now subjected to a salt spray test as specified in ASTM B 117 of a duration of 600 hours. After every 200 hours weathering, the state of the coating is assesed, specifically the degree of bubbling (as specified in DIN 53,209) at the cross-cut and on the painted surface and also the degree of rusting (as specified in DIN 53,210) on the whole surface.

At the end of the tests, the coating is removed by treatment with concentrated sodium hydroxide solution, and the corrosion of the metal at the cross-cut (as specified in DIN 53,167) and over the remainder of the surface is assessed. In each case the assessment is carried out in accordance with a 6-point scale. The sum of the assessment of the coating and the assessment of the metal surface gives the anti-corrosion value AC. The higher this is the more effective is the inhibitor tested.

Table II

Results of the Salt Spray Test

| Example | Corrosion Inhibitor | Amount added | Assessment of coating | Assessment of metal | AC |
|---|---|---|---|---|---|
| - | None | - | 2.2 | 0.6 | 2.8 |
| 27 | Product of Example 1 | 2 %<br>4 % | 5.0<br>5.4 | 5.2<br>5.0 | 10.2<br>10.4 |
| 28 | Product of Example 2 | 2 %<br>4 % | 2.2<br>2.4 | 4.0<br>4.2 | 6.2<br>6.6 |
| 29 | Product of Example 4 | 2 % | 4.1 | 4.3 | 8.4 |
| 30 | Product of Example 5 | 2 %<br>4 % | 4.9<br>3.9 | 3.7<br>4.0 | 8.6<br>7.9 |
| 31 | Product of Example 6 | 2 %<br>4 % | 4.8<br>3.7 | 4.7<br>3.7 | 9.5<br>7.4 |
| 32 | Product of Example 7 | 2 % | 2.5 | 3.2 | 5.7 |
| 33 | Product of Example 8 | 2 % | 2.7 | 2.9 | 5.6 |
| 34 | Product of Example 9 | 2 % | 1.8 | 1.7 | 3.5 |
| 35 | Product of Example 10 | 2 % | 2.4 | 3.8 | 6.2 |
| 36 | Product of Example 11 | 2 % | 1.8 | 1.3 | 3.1 |
| 37 | Product of Example 12 | 2 % | 3.4 | 2.5 | 5.9 |

Table II (continuation)

Results of the Salt Spray Test

| Example | Corrosion Inhibitor | Amount added | Assessment of coating | Assessment of metal | AC |
|---|---|---|---|---|---|
| 38 | Product of Example 13 | 2 % | 2.6 | 2.5 | 5.1 |
| 39 | Product of Example 14 | 2 % | 3.8 | 4.0 | 7.8 |
| 40 | Product of Example 15 | 2 % | 3.7 | 2.9 | 6.6 |
| 41 | Product of Example 16 | 2 % | 3.3 | 2.8 | 6.1 |
| 42 | Product of Example 17 | 2 % | 2.2 | 3.9 | 6.1 |
| 43 | Product of Example 18 | 2 % | 3.8 | 4.0 | 7.8 |
| 44 | Product of Example 19 | 2 % | 3.5 | 2.6 | 6.1 |
| 45 | Product of Example 20 | 2 % | 3.0 | 1.9 | 4.9 |
| 46 | Product of Example 21 | 2 % | 2.8 | 2.7 | 5.5 |
| 47 | Product of Example 22 | 2 % | 2.9 | 1.5 | 4.4 |
| 48 | Product of Example 23 | 2 % | 4.8 | 4.7 | 9.5 |
| 49 | Product of Example 24 | 2 % | 2.0 | 2.5 | 4.5 |
| 50 | Product of Example 25 | 2 % | 2.4 | 4.1 | 6.5 |
| 51 | Product of Example 26 | 2 % | 1.9 | 2.6 | 4.5 |
| 52 | Product of Example 27 | 2 % | 4.9 | 3.7 | 8.6 |

## Claims

1. A composition comprising a coating material and at least one compound having the formula I:

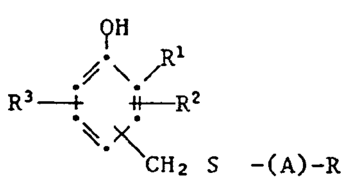

I

wherein $R^1$ and $R^2$ may be the same or different and each is hydrogen, $C_1$-$C_{15}$alkyl, $C_3$-$C_{15}$alkenyl, $C_7$-$C_{10}$phenylalkyl, $C_6$-$C_{18}$aryl having 6 or 10 aromatic ring carbon atoms; or $C_5$-$C_{12}$cycloalkyl;
$R^3$ is hydrogen, $C_1$-$C_{15}$alkyl or $C_2$-$C_{15}$alkenyl;
A is a residue of a heterocyclic ring having 5-7 ring members and 1-4 nitrogen atoms, or A is a residue of a heterocyclic ring having 5-7 ring memberes and 1-4 nitrogen atoms and is fused to a further heterocyclic ring or to a carbocyclic ring,

and wherein R is one or more of hydrogen, $C_1$-$C_{15}$alkyl, $C_1$-$C_4$halogenoalkyl, $C_1$-$C_{12}$alkoxy, $C_1$-$C_{12}$alkylthio, phenylthio, benzylthio, $C_1$-$C_{12}$alkylsulphonyl, phenyl, $C_7$-$C_{15}$alkylphenyl, $C_7$-$C_{10}$phenylalkyl, $C_5$-$C_8$cycloalkyl, halogen, $NO_2$, CN, COOH, COO($C_1$-$C_{12}$alkyl), OH, $NH_2$, $CONH_2$, $NHR^4$, $N(R^4)_2$, $CONH(R^4)$ or $-CON(R^4)_2$ wherein $R^4$ is $C_1$-$C_{12}$alkyl, $C_3$-$C_{12}$alkyl which is interrupted by one or more O-atoms, $C_5$-$C_8$cycloalkyl, benzyl, phenyl or phenyl which is substituted by halogen, $C_1$-$C_4$alkyl, $C_1$-$C_4$alkoxy or $NO_2$, or $N(R^4)_2$ is a pyrrolidino, piperidino or morpholine group; provided that A is not benzothiazole residue.

2. A composition according to claim 1 wherein R-(A)- is a residue in which one R is hydrogen, $C_1$-$C_4$alkyl, $C_1$-$C_4$alkoxy, trifluoromethoxy, halogen or nitro and other groups R are hydrogen; $R^1$ is $C_1$-$C_8$alkyl, $C_7$-$C_{10}$phenylalkyl, phenyl or cyclohexyl; $R^2$ is hydrogen, $C_1$-$C_8$alkyl, $C_7$-$C_{10}$phenylalkyl, phenyl or cyclohexyl; and $R^3$ is hydrogen or $C_1$-$C_4$alkyl.

3. A composition according to claim 1 wherein the compound of formula I is a compound having the formula IA:

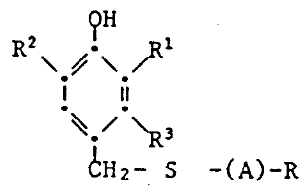

IA

wherein R, $R^1$, $R^2$, $R^3$, n and A are as defined in claim 1.

4. A composition according to claim 3 wherein $R^1$ and $R^2$, independently, are hydrogen, $C_1$-$C_{12}$alkyl, $C_7$-$C_{10}$phenylalkyl, phenyl or cyclohexyl.

5. A composition according to claim 4 wherein $R^1$ and $R^2$, are $C_1$-$C_5$alkyl and $R^3$ is hydrogen.

6. A composition according to claim 1 wherein the compound of formula I is a compound having the formula IB:

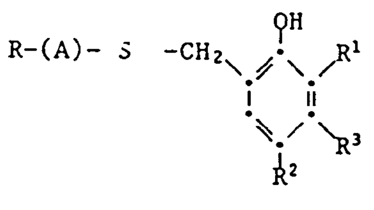

IB

wherein R, $R^1$, $R^2$, $R^3$, n and A are as defined in claim 1.

7. A composition according to claim 6 wherein $R^1$ is $C_1$-$C_8$alkyl, $C_1$-$C_4$alkoxy, $C_7$-$C_{10}$phenylalkyl, phenyl or cyclohexyl, $R^2$ is hydrogen $C_1$-$C_8$alkyl, $C_7$-$C_{10}$phenylalkyl, phenyl or cyclohexyl.

8. A composition according to claim 7 wherein $R^1$ and $R^2$ are each $C_1$-$C_5$alkyl.

9. A composition according to claim 1 wherein the coating material is a primer for a metallic substrate.

10. A composition according to claim 9 wherein the metallic substrate is iron, steel, copper, zinc and aluminium.

11. A composition according to claim 1 wherein the coating material is an aqueous coating material.

12. A composition according to claim 11 wherein the aqueous paint is a cathodically-depositable coating material.

13. A composition according to claim 1 wherein the coating material contains, based on the solids content of the coating material, 0.5 to 5 wt % of at least one compound of formula I as defined in claim 1.

14. A composition according to claim 1 wherein the coating material is based on an epoxy resin, a polyurethane, an aminoplast, an acrylic-, alkyd- or polyester resin, or a mixture of such resins.

15. A composition according to claim 1 wherein the coating material is based on a vinyl polymer, a cellulose ester, a chloro-rubber, a phenol resin, a styrene-butadiene copolymer or a drying oil.

16. A compound having the formula I':

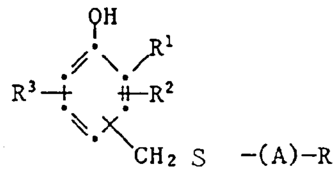

I'

wherein R, $R^1$, $R^2$, $R^3$, n and A are as defined in claim 1 provided that:

A is not benzothiazole residue; and when

A is a heterocyclic residue having 5-7 ring members and 1-4 nitrogen atoms, $R^2$ is H, $R^1$ and $R^3$ are each tert.-butyl and $R^3$ is in ortho-position to the -OH group, then A is not pyridino-thio group.

17. A method of making a coating material having improved corrosion inhibition properties and antioxidation properties, comprising the step of adding to said-coating material an effective amount of a compound of formula I according to claim 1.

**Patentansprüche**

1. Zusammensetzung, umfassend ein Überzugsmaterial und mindestens eine Verbindung der Formel I:

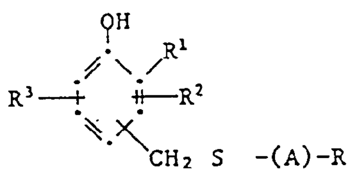

I

worin $R^1$ und $R^2$ gleich oder verschieden sein können und jeweils Wasserstoff, $C_1$-$C_{15}$-Alkyl, $C_3$-$C_{15}$-Alkenyl, $C_7$-$C_{10}$-Phenylalkyl, $C_6$-$C_{18}$-Aryl mit 6 oder 10 aromatischen Ringkohlenstoffatomen oder $C_5$-$C_{12}$-Cycloalkyl bedeuten; $R^3$ Wasserstoff, $C_1$-$C_{15}$-Alkyl oder $C_2$-$C_{15}$-Alkenyl bedeutet; A einen Rest eines heterocyclischen Rings mit 5 bis 7 Ringgliedern und 1 bis 4 Stickstoffatomen darstellt oder A einen Rest eines heterocyclischen Rings mit 5 bis 7 Ringgliedern und 1 bis 4 Stickstoffatomen bedeutet und an einen weiteren heterocyclischen oder carbocyclischen Ring kondensiert ist und worin R eines oder mehrere von Wasserstoff, $C_1$-$C_{15}$-Alkyl, $C_1$-$C_4$-Halogenalkyl, $C_1$-$C_{12}$-Alkoxy, $C_1$-$C_{12}$-Alkylthio, Phenylthio, Benzylthio, $C_1$-$C_{12}$-Alkylsulfonyl, Phenyl, $C_7$-$C_{15}$-Alkylphenyl, $C_7$-$C_{10}$-Phenylalkyl, $C_5$-$C_8$-Cycloalkyl, Halogen, $NO_2$, CN, COOH, COO($C_1$-$C_{12}$-Alkyl), OH, $NH_2$, $CONH_2$, $NHR^4$, $N(R^4)_2$, $CONH(R^4)$ oder $-CON(R^4)_2$ bedeutet, worin $R^4$ $C_1$-$C_{12}$-Alkyl, $C_3$-$C_{12}$-Alkyl unterbrochen mit einem oder mehreren Sauerstoffatomen, $C_5$-$C_8$-Cycloalkyl, Benzyl, Phenyl oder Phenyl substituiert mit Halogen, $C_1$-$C_4$-Alkyl, $C_1$-$C_4$-Alkoxy oder $NO_2$ bedeutet oder $N(R^4)_2$ eine Pyrrolidino, Piperidino oder Morpholinogruppe darstellt mit der Maßgabe, daß A kein Benzothiazolrest ist.

2. Zusammensetzung nach Anspruch 1, wobei R-(A)- ein Rest ist, in dem der Rest R Wasserstoff, $C_1$-$C_4$-Alkyl, $C_1$-$C_4$-Alkoxy, Trifluormethoxy, Halogen oder Nitro bedeutet und die anderen Reste R Wasserstoff

sind; $R^1$ $C_1$-$C_8$-Alkyl, $C_7$-$C_{10}$-Phenylalkyl, Phenyl oder Cyclohexyl bedeutet; $R^2$ Wasserstoff, $C_1$-$C_8$-Alkyl, $C_7$-$C_{10}$-Phenylalkyl, Phenyl oder Cyclohexyl bedeutet; und $R^3$ Wasserstoff oder $C_1$-$C_4$-Alkyl darstellt.

3. Zusammensetzung nach Anspruch 1, wobei die Verbindung der Formel I eine Verbindung der Formel IA ist:

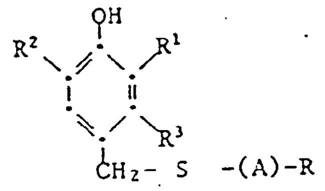

IA

worin R, $R^1$, $R^2$, $R^3$, n und A wie in Anspruch 1 definiert sind.

4. Zusammensetzung nach Anspruch 3, wobei $R^1$ und $R^2$ unabhängig voneinander Wasserstoff, $C_1$-$C_{12}$-Alkyl, $C_7$-$C_{10}$-Phenylalkyl, Phenyl oder Cyclohexyl darstellen.

5. Zusammensetzung nach Anspruch 4, wobei $R^1$ und $R^2$ $C_1$-$C_5$-Alkyl darstellen und $R^3$ Wasserstoff ist.

6. Zusammensetzung nach Anspruch 1, wobei die Verbindung der Formel I eine Verbindung der Formel IB ist:

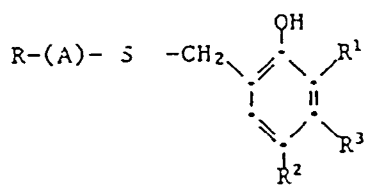

IB

worin R, $R^1$, $R^2$, $R^3$, n und A wie in Anspruch 1 definiert sind.

7. Zusammensetzung nach Anspruch 6, wobei $R^1$ $C_1$-$C_8$-Alkyl, $C_1$-$C_4$-Alkoxy, $C_7$-$C_{10}$-Phenylalkyl, Phenyl oder Cyclohexyl bedeutet; $R^2$ Wasserstoff, $C_1$-$C_8$-Alkyl, $C_7$-$C_{10}$-Phenylalkyl, Phenyl oder Cyclohexyl darstellt.

8. Zusammensetzung nach Anspruch 7, wobei $R^1$ und $R^2$ jeweils $C_1$-$C_5$-Alkyl sind.

9. Zusammensetzung nach Anspruch 1, wobei das Überzugsmaterial ein Grundiermittel für ein metallisches, Substrat ist.

10. Zusammensetzung nach Anspruch 9, wobei das metallische Substrat Eisen, Stahl, Kupfer, Zink und Aluminium ist.

11. Zusammensetzung nach Anspruch 1, wobei das Überzugsmaterial ein wässeriges Überzugsmaterial ist.

12. Zusammensetzung nach Anspruch 11, wobei der wässerige Anstrichstoff ein kathodisch. abscheidbares Überzugsmaterial ist.

13. Zusammensetzung nach Anspruch 1, wobei das Überzugsmaterial, bezogen auf den Feststoffgehalt des Überzugsmaterials, 0,5 bis 5 Gew.-% mindestens einer Verbindung der Formel I gemäß Anspruch 1 enthält.

14. Zusammensetzung nach Anspruch 1, wobei das Überzugsmaterial auf einem Epoxidharz, einem Polyurethan, einem Aminoplast, einem Acryl-, einem Alkyd- oder Polyesterharz oder einem Gemisch solcher Harze basiert.

15. Zusammensetzung nach Anspruch 1, wobei das Überzugsmaterial auf einem Vinylpolymer, einem Celluloseester, einem Chlorkautschuk, einem Phenolharz, einem Styrol/Butadien-Copolymer oder einem trocknenden Öl basiert.

**16.** Verbindung der Formel I':

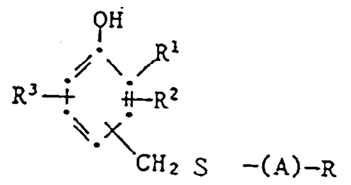

I'

worin R, $R^1$, $R^2$, $R^3$, n und A wie in Anspruch 1 definiert sind, mit der Maßgabe, daß A kein Benzothiazolrest ist und wenn A ein heterocyclischer Rest mit 5-7 Ringgliedern und 1-4 Stickstoffatomen ist, $R^2$ H ist, $R^1$ und $R^3$, jeweils tert.Butyl darstellen und $R^3$ sich in ortho-Stellung zur OH-Gruppe befindet, dann bedeutet A keine Pyridinothiogruppe.

**17.** Verfahren zur Herstellung eines Überzugsmaterials mit verbesserten Korrosionsinhibierungseigenschaften und Antioxidationseigenschaften, umfassend den Schritt der Zugabe einer wirksamen Menge einer Verbindung der Formel I gemäß Anspruch 1 zu dem Überzugsmaterial.

## Revendications

**1.** Une composition comprenant une matière de revêtement avec un ou plusieurs composés de formule I cidessous :

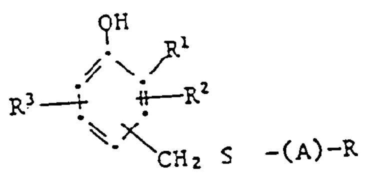

(I)

dans laquelle $R^1$ et $R^2$, qui peuvent être identiques ou différents l'un de l'autre, sont chacun l'hydrogène, un alkyle en $C_1$-$C_{15}$, un alcényle en $C_3$-$C_{15}$, un phénylalkyle en $C_7$-$C_{10}$, un aryle en $C_6$-$C_{18}$ avec 6 ou 10 atomes de carbone de cycle aromatique ou encore un cycloalkyle en $C_5$-$C_{12}$ ;
$R^3$ représente l'hydrogène, un alkyle en $C_1$-$C_{15}$ ou un alcényle en $C_2$-$C_{15}$ ;
A le radical d'un composé hétérocyclique avec de 5 à 7 chaînons du cycle et de 1 à 4 atomes d'azote, ou bien le radical d'un tel composé hétérocyclique condensé avec un autre noyau hétérocyclique ou carbocyclique, et
R représente un ou plusieurs radicaux pris parmi l'hydrogène, des alkyles en $C_1$-$C_{15}$, halogénoalkyles en $C_1$-$C_4$, alcoxy en $C_1$-$C_{12}$, alkylthio en $C_1$-$C_{12}$, phénylthio, benzylthio, alkylsulfonyles en $C_1$-$C_{12}$, phényles, alkylphényles en $C_7$-$C_{15}$, phénylalkyles en $C_7$-$C_{10}$, cycloalkyles en $C_5$-$C_8$, des halogènes et les groupes $NO_2$, CN, COOH, COO($C_1$-$C_{12}$alkyles), OH, $NH_2$, $CONH_2$, $NHR^4$, $N(R^4)_2$, $CONH(R^4)$ et -$CON(R^4)_2$, $R^4$ désignant un alkyle en $C_1$-$C_{12}$, un alkyle en $C_3$-$C_{12}$ interrompu par un ou plusieurs atomes d'oxygène, un cycloalkyle en $C_5$-$C_8$, un benzyle, un phényle ou un halogénophényle ou encore un phényle avec un alkyle en $C_1$-$C_4$, un alcoxy en $C_1$-$C_4$ ou le groupe $NO_2$, ou bien $N(R^4)_2$ représente un groupe pyrrolidino, piperidino ou morpholino ; avec la condition que A ne soit pas un radical de benzothiazole.

**2.** Une composition selon la revendication 1 caractérisée en ce que dans le radical R-(A)- de la formule I un R est l'hydrogène, un alkyle ou un alcoxy en $C_1$-$C_4$, le groupe trifluorométhoxy, un halogène ou le groupe nitro et les autres R sont l'hydrogène ; $R^1$ est un alkyle en $C_1$-$C_8$, un phénylalkyle en $C_7$-$C_{10}$, un phényle ou un cyclohexyle ; $R^2$ l'hydrogène, un alkyle en $C_1$-$C_8$, un phénylalkyle en $C_7$-$C_{10}$, un phényle ou un cyclohexyle ; et $R^3$ l'hydrogène ou un alkyle en $C_1$-$C_4$.

**3.** Une composition selon la revendication 1 dont le composé de formule I est un composé de formule IA :

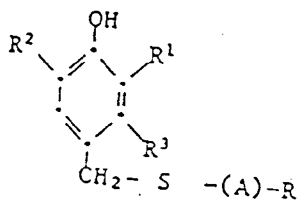

(IA)

les divers symboles ayant les significations données à la revendication 1.

**4.** Une composition selon la revendication 3 caractérisée en ce que dans la formule IA, $R^1$ et $R^2$ sont chacun, indépendamment l'un de l'autre, l'hydrogène, un alkyle en $C_1$-$C_{12}$, un phénylalkyle en $C_7$-$C_{10}$, un phényle ou un cyclohexyle.

**5.** Une composition selon la revendication 4 caractérisée en ce que $R^1$ et $R^2$ sont des alkyles en $C_1$-$C_5$ et $R^3$ est l'hydrogène.

**6.** Une composition selon la revendication 1 dans laquelle le composé de formule I est un composé de formule IB :

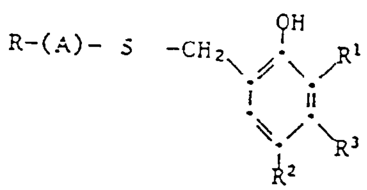

(IB)

les divers symboles ayant les significations données à la revendication 1.

**7.** Une composition selon la revendication 6 caractérisée en ce que dans la formule IB $R^1$ est un alkyle en $C_1$-$C_8$, un alcoxy en $C_1$-$C_4$, un phénylalkyle en $C_7$-$C_{10}$, un phényle ou un cyclohexyle, et $R^2$ l'hydrogène, un alkyle en $C_1$-$C_8$, un phénylalkyle en $C_7$-$C_{10}$, un phényle ou un cyclohexyle.

**8.** Une composition selon la revendication 7 caractérisée en ce que $R^1$ et $R^2$ sont chacun un alkyle en $C_1$-$C_5$.

**9.** Une composition selon la revendication 1 dont la matière de revêtement est un apprêt ou couche de fond pour substrats métalliques.

**10.** Une composition selon la revendication 9 pour laquelle le substrat métallique est du fer, de l'acier, du cuivre, du zinc ou de l'aluminium.

**11.** Une composition selon la revendication 1 dans laquelle la matière de revêtement est une matière aqueuse.

**12.** Une composition selon la revendication 11 dans laquelle la peinture aqueuse est une matière de revêtement déposable par voie cathodique.

**13.** Une composition selon la revendication 1 qui comprend, par rapport à la teneur en matières solides de la matière de revêtement, de 0,5 à 5 % en poids d'un ou de plusieurs composés de formule I selon la revendication 1.

**14.** Une composition selon la revendication 1 dont la matière de revêtement est à base d'une résine époxyde, d'un polyuréthanne, d'un aminoplaste, d'une résine acrylique ou alkyde ou de polyester, ou de plusieurs de ces résines ensemble.

**15.** Une composition selon la revendication 1 dont la matière de revêtement est à base d'un polymère vinylique, d'un ester de cellulose, d'un caoutchouc chloré, d'une résine phénolique, d'un copolymère de styrène et butadiène ou d'une huile siccative.

**16.** Les composés de formule I' ci-dessous :

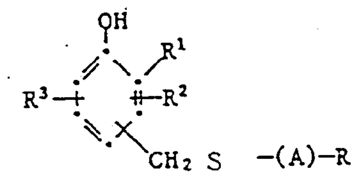

(I')

dont les divers symboles ont les significations données à la revendication 1, avec la condition que A ne soit pas un radical de benzothiazole ; et si A est un radical hétérocyclique avec de 5 à 7 chaînons de cycle et de 1 à 4 atomes d'azote, $R^2$ l'hydrogène, $R^1$ et $R^3$ chacun le groupe tert-butyle et $R^3$ occupe la position ortho par rapport au groupe -OH, A n'est pas un groupe pyridino-thio.

**17.** Une méthode de préparation de produits de revêtement ayant une action d'inhibition de la corrosion améliorée et aussi un effet contre l'oxydation amélioré, méthode selon laquelle on ajoute à la matière de revêtement une proportion efficace d'un ou de plusieurs composés de la revendication 1.